# NEW EUROPEAN PATENT SPECIFICATION

(11) **EP 1 475 060 B2**
(45) Date of publication and mention of the opposition decision: **23.09.2009**
(45) Mention of the grant of the patent: 05.04.2006
(21) Application number: 04101985.2
(22) Date of filing: 07.05.2004
(51) Int. Cl.: A61F 5/01

(54) **Articulated joint for postoperative brace**
Gelenkverbindung für eine postoperative Orthese
Joint articulé pour une orthèse post-opératoire

(30) Priority: 09.05.2003 IT VR20030057
(43) Date of publication of application: 10.11.2004
(73) Proprietor: F.G.P. Srl, 37062 Dossobuono (VR) (IT)
(72) Inventor: Turrini, Alberto, 37060, Castel D'Azzano (IT); Ferrigolo, Moreno, 37062, DOSSOBUONO (VR) (IT)
(74) Representative: Ferro, Stefano

(56) References cited:
- EP-A- 1 086 672
- EP-A- 1 086 672
- WO-A-02/02035
- WO-A-02/02035
- US-A- 5 814 000
- US-A- 5 827 208

## Description

### TECHNICAL FIELD

This invention concerns an articulated joint for a postoperative brace.

More specifically, this invention refers to an articulated joint for a brace, for example a knee brace or a walker, equipped with end of stroke adjustment to control the angular movement both in extension and in flexion.

This joint can be used for pre- and postoperative post-trauma therapy.

This invention can be applied in the medical industry with particular reference to manufacturers of prostheses and braces.

### BACKGROUND ART

The use is known of articulated joints for knee braces or walkers equipped with a device for adjustment of the angular movement to ensure correct end of stroke both in extension and flexion of the tibia with respect to the femur.

According to a known technique, an articulated joint for a knee brace consists of a pair of reciprocally hinged joints, each integral with a respective upright fixed in turn to the limb of the user.

In post-trauma therapy it is indispensable that the angular movement between one disc and the other is limited within a range that gradually increases in relation to time; different control must also be ensured for the angular movement in extension with respect to the movement necessary for flexion

The adjustment of these angular movements must be carried out by specialised personnel or, according to precise indications provided by the doctor, by the user who can adjust the device directly.

The articulated joint must therefore be provided with adjustment devices that are easily accessible as well as being easy to manipulate.

Various articulated joints for knee braces with angular movement adjustment devices have already been proposed.

Some such articulated joints foresee the use of discs equipped with a plurality of threaded holes around the circumference; threaded pins can be screwed into these holes to define the end of stroke.

The extraction of these pins and their subsequent reinsertion in other holes makes it possible to change the angular movement allowed between the two discs.

The removal and subsequent reinsertion of the pins, which present threadings with a relatively limited diameter, has been found to be an operation that is not easy for the user to carry out, making the articulated joint difficult to manage.

One solution was proposed in the document US-A-401933 foreseeing the use of non-threaded pins. These pins present a smooth surface, making them easier to insert and extract from the respective holes in the joint. The pins are temporarily fixed inside the respective holes thanks to the presence of a flat spring, constrained to each of the pins and designed to guide them according to a direction parallel to the axis of rotation of the articulated joint.

However, in this case too a certain dexterity is required to move the spring away from the body of the joint and to extract the pin from its respective hole.

Another solution was proposed in the document US-A-5672152 which describes an articulated joint consisting of an inner and an outer disc aligned parallely and rotationally connected by means of a central common pin.

The:perimeter of the outer disc presents a plurality of notches, while the inner disc presents a slot in which a retainer equipped with elastic loading means can be inserted.

When in use, the retainer is simultaneously inserted in a notch in the outer disc thus ensuring an end of stroke for the relative angular movement of the two discs.

The elastic loading means consists of a curved metal wire whose flexibility ensured adequate elastic return.

The user can adjust the articulated joint by means of the retainer and the metal wire, intervening in the direction of traction of the elastic loading means towards the outside and releasing it in the required position corresponding to another notch in the outer disc.

While partially solving the problems described above, this solution is not without some limitations.

In fact, moving the retainer outwards and then releasing it for elastic return is still an operation that requires a certain ability.

Another drawback is represented by the fact that the elastic loading means does not present a high degree of reliability, being excessively simplified in that it consists of a single section of curved metal wire.

A further solution, foreseen in the description of the document US-A-5827208, presents an articulated joint similar to the one described above with the addition of an intermediate disc equipped with variously shaped openings and designed to accommodate a respective pawl. In this case, while the articulated joint is more manageable, it is also much more complex, making it difficult to produce and therefore economically disadvantageous.

Document EP-A-1086672 discloses an orthopedic brace having a range of motion hinge with radially actuated stops. The hinge provides for selective adjustment of both a flexion angle or a range of motion and an extension angle or range of motion about an axis of the hinge assembly which typically corresponds to a joint axis of the user. The adjustment of the flexion and extension angles is accomplished by radially actuated stops. The stops are positively biassed into one of a plurality of selective positions (one set for flexion and one for extension) and disengageable by the user to set the respective range of motion of the struts.

### DESCRIPTION OF THE INVENTION

This invention proposes to provide an articulated joint for a postoperative knee brace that can eliminate or significantly reduce the disadvantages described above.

This invention also proposes to provide a safe and reliable articulated joint for the therapy of patients following trauma to the knee cap.

This is achieved by means of an articulated joint for a postoperative knee brace with the features described in the main claim.

The dependent claims describe advantageous embodiments of the invention.

The articulated joint for a postoperative knee brace according to the invention comprises at least one inner disc-shaped element, equipped with blocking teeth and integral with a first upright, for example a tibial upright, designed to be accommodated within a space delimited by a pair of outer disc-shaped elements, integral with a second upright, for example a femoral upright, and hinged to the inner disc-shaped element, each outer disc-shaped element being equipped with at least one toothed circumferential slot designed to house a gudgeon pin integral with a strip which can be moved radially and is free to swivel inside the outer disc-shaped elements, presenting elastic loading means positioned between the gudgeon pin and the central rotation pin of the articulated joint.

According to the invention, the articulated joint presents an inner circumferential slot, with respect to the reciprocal swivel of the two uprights, for adjustment of the angle of flexion, and a smaller outer circumferential slot, for adjustment of the angle of extension.

The articulated joint comprises a strip and a gudgeon pin for each slot.

Each strip presents an opening in which the central pin of the articulated joint is slidably housed.

When in use, the free longitudinal end of the strip protrudes from the outer discs and advantageously presents a rough surface so that it can be easily pressed by a user.

The elastic loading means, consisting for example of a helical spring, presents one end that is indirectly attached to the central pin of the articulated joint and the other end attached to the part of the strip to which the gudgeon pin is directly connected.

The means of elastic loading can be advantageously inserted in an appropriate housing cut in the body of the strip.

Each means of elastic loading tends to push the respective gudgeon pin radially outwards, this pin being housed in a notch in the respective toothed slot and designed to act as an end of stroke for the angular movement of the inner disc-shaped element with respect to the outer disc-shaped elements.

In fact, at the end of the allowed swivel, the teeth of the inner disc-shaped element strike the gudgeon pin, consequently limiting the angular movement of the tibial upright with respect to the femoral upright.

The independent movement of the gudgeon pins inside other notches available in the respective circumferential slots ensures a different angular movement and thus an adjustment of the flexion angle and the extension angle.

According to this invention, this movement can be easily performed by the user by simply pressing the protruding end of the strip. This action causes compression of the elastic loading means and the consequent release of the gudgeon pin from the occupied notch.

The user can then pull the strip into a new position and, when the free end of the strip is released, the contrast force of the elastic loading means ensures that the gudgeon pin is housed in another notch available in the respective circumferential slot.

Since these operations require mere pressure on and movement of the strip, the adjustment of the articulated joint according to the invention is extremely simple and easy without having to resort to the assistance of specialised personnel.

The articulated joint can be made from lightweight metal alloy or high-resistance composite plastic material.

### DESCRIPTION OF THE DRAWINGS

Other features and advantages of the invention will become evident on reading the following description of an embodiment of the invention, provided as a non-binding example, with the help of the drawings on the attached pages, in which:
- figure 1 is a prospective view slightly from above of an articulated joint according to the invention; and
- figure 2 is a prospective view slightly from above, on an enlarged scale, of the articulated joint shown in figure 1 without one of the outer disc elements and one of the inner disc elements.

### DESCRIPTION OF AN EMBODIMENT

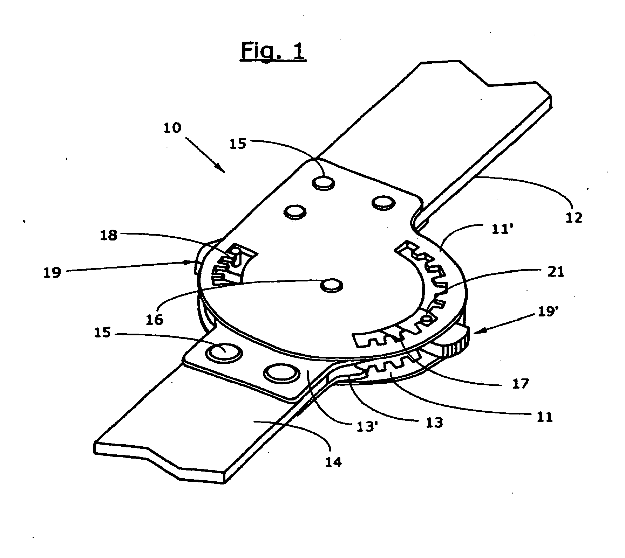

Referring first to figure 1, the reference number 10 indicates in general an articulated joint, in this case applied to a knee brace, equipped with end of stroke adjustment to control the angular movement both in extension and flexion.

The articulated joint 10 comprises a pair of substantially disc-shaped outer elements 11, 11' integral with a femoral upright 12 and delimiting an inner space designed to accommodate at least one inner disc-shaped element 13, in turn integral with a tibial upright 14.

Figure 1 shows two inner disc-shaped elements 13, 13'.

According to particularly advantageous embodiment of the invention, the outer disc-shaped elements 11, 11' and the inner disc-shaped elements 13, 13' are respectively fixed to the femoral upright 12 and to the tibial upright 14 by means of punch couplings 15.

The outer disc-shaped elements 11, 11' and the inner disc-shaped elements 13, 13' are hinged by means of a common central pin 16 of the joint 10 so as to allow reciprocal angular movements to be carried out.

On the side portions distal to the couplings 15, each outer disc-shaped element 11 presents a first circumferential toothed slot 17, with a relatively large angular extension, and a second circumferential toothed slot 18, with a relatively small angular extension.

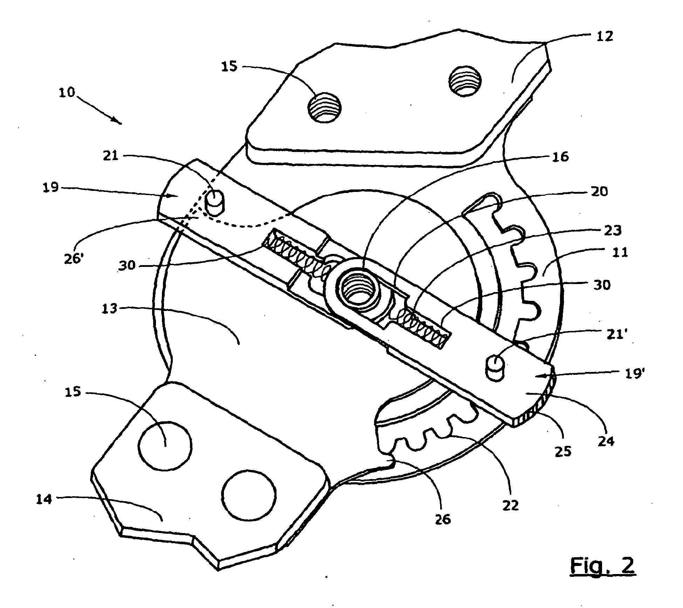

As can be seen in figure 2, two strips 19, 19' are located in a central position, arranged radially and opposite each other in correspondence with the circumferential slots 17, 18, these strips 19 being mounted to rotate around the central pin 16.

Each strip 19, 19' presents a longitudinal opening 20 designed to slidingly house the central pin 16 in order to allow a radial movement of the strip 19, 19'.

The portion of each strip 19, 19' radially distal from the pin 16 presents a gudgeon pin 21, 21' designed to be accommodated in a notch 22 of a respective circumferential slot 17, 18.

Each strip 19, 19' also comprises a means of elastic loading, for example a spring 23, positioned between the gudgeon pin 21 and the central pin 16 of the articulated joint 10. The means of elastic loading can be advantageously inserted in an appropriate housing 30 cut in the body of the strip 19, 19'.

According to the invention, as shown in figure 2, the spring 23 presents one end 16 attached to the outer end of the opposite strip while the other end of the spring is attached to the part connected to the gudgeon pin 21 of the strip 19 in which the spring is housed 23.

The free end 24 of each strip 19, 19' presents a rough surface 25, for example knurled, in order to favour the manual grip on the strips 19.

Each inner disc-shaped element 13, 13' presents teeth 26 designed to strike against the respective gudgeon pins 21 ensuring end of stroke for the angular movement allowed between the inner disc-shaped element 13 and the outer disc-shaped elements 11, 11'.

The inner circumferential slot 17, for adjustment of the flexion angle, can present an angular extension of between 10 and 120 sexagesimal degrees, while the outer circumferential slot 18, for adjustment of the extension angle, can have a smaller angular extension, generally not more than 30 sexagesimal degrees.

The articulated joint 10 can be made from lightweight metal alloy or high-resistance composite plastic material.

According to an advantageous embodiment of the invention, not shown in the figures, one of the outer disc-shaped elements is fitted with a protective transparent cover, which is also fitted to rotate around the central pin of the articulated joint.

The outer portion of the protective cover presents a pair of opposite openings that make it possible to manually activate the strips 19, 19'; once the opening of the articulated joint has been manually adjusted, the protective cover is rotated so that the strips cannot be operated manually, by mistake or as a result of a knock.

The invention is described above with reference to a particular embodiment, applied to a knee brace. It is nevertheless clear that the invention can also be applied to post-operative braces of any type, in particular to walkers.

## Claims

1. An articulated joint (10) for a post-operative brace comprising at least one inner disc-shaped element (13; 13'), equipped with blocking teeth (26, 26') and integral with a first upright (14), the inner disc-shaped element being designed to be accommodated inside a space delimited by a pair of outer disc-shaped elements (11: 11'), integral with a second upright (12) and hinged to the inner disc-shaped elements (13, 13'), each outer disc-shaped element (11) being equipped with a pair of toothed circumferential slots (17, 18) designed to accommodate blocking means (21, 21') which are integral with a strip (19, 19'), which can be moved radially and is free to swivel inside the outer disc-shaped elements (11), wherein the blocking means consists of at least one respective gudgeon pin (21, 21'), the strips (19, 19') presenting a means of elastic loading (23) positioned between the respective gudgeon pin (21; 21') forming said blocking means and a central rotation pin (16) of the articulated joint (10), **characterised in that** said elastic loading means (23) presents one end attached to the outer end of the opposite strip (19, 19') anchored to the central rotation pin (16) while the other end of the elastic loading means (23) is attached to the part connected to the gudgeon pin (21, 21') of the strip (19, 19') in which the means of elastic loading (23) is housed.

2. An articulated joint (10) according to claim 1, **characterised in that** it comprises a first inner circumferential slot (17), for adjustment of the flexion angle, and a second outer circumferential slot (18), for adjustment of the extension angle.

3. An articulated joint (10) according to any of the foregoing claims, **characterised in that** each strip (19, 19') presents an opening (20) designed to slidingly housed the central pin (16).

4. An articulated joint (10) according to any of the foregoing claims, **characterised in that** the free longitudinal end (24) of the strip (19, 19'), protruding from the outer disc-shaped elements (11, 11'), presents a rough surface (25), advantageously knurled.

5. An articulated joint (10) according to any of the foregoing claims, **characterised in that** the elastic loading means (23) consists of a spring.

6. An articulated joint (10) according to any of the foregoing claims, **characterised in that** the elastic loading means (23) is inserted in a housing (30) cut in the body of the strip (19, 19').

7. An articulated joint (10) according to any of the foregoing claims, **characterised in that** each circumferential slot (17, 18) presents notches (22) arranged radially in the outer portion of each circumferential slot (17, 18).

8. An articulated joint (10) according to any of the foregoing claims, **characterised in that** it is made from lightweight metal alloy and/or high-resistance composite plastic material.

9. An articulated joint (10) according to any of the foregoing claims, **characterised in that** it also comprises a protective cover, advantageously made from transparent material.

10. An articulated joint (10) according to claim 9, **characterised in that** the protective cover is mounted to rotate around the central pin of the articulated joint.

11. An articulated joint (10) according to claim 9 or 10, **characterised in that** the protective cover presents a pair of opposite side openings that allow the manual activation of the strips (19, 19'), the rotation of this protective cover causing the inaccessibility of the strips (19, 19') so that they cannot be moved manually, by mistake or as a result of a knock.

12. An articulated joint (10) according to any of the foregoing claims, **characterised in that** it is applied to a knee brace or to a post-operative walker.

## Patentansprüche

1. Gegliedertes Scharnier (10) für eine postoperative Schiene, bestehend aus mindestens einem mit Blockierzähnen (26, 26') versehenen, inneren scheibenförmigen Element (13, 13'), das integriert mit einem ersten Vertikalteil (14) ausgebildet ist, wobei das innere, scheibenförmige Element, so ausgeführt ist, dass es im Inneren eines von einem Paar äußerer scheibenförmiger Elemente (11, 11') umschlossenen Hohlraumes aufgenommen wird, mit einem zweiten Vertikalteil integriert und an die inneren scheibenförmigen Elemente (13, 13') angelenkt ist, wobei jedes äußere scheibenförmige Element (11) ein Paar verzahnte Rundschlitze (17, 18) aufweist, die so ausgeführt sind, dass sie ein Blockiermittel (21, 21') aufnehmen, welches so ausgeführt ist, dass es mit einer radial bewegbaren, in den äußeren scheibenförmigen Elementen (11) frei schwenkbaren Leiste (19, 19') integriert ist, wobei das Blockiermittel aus mindestens einem Anschlagstift (21. 21') besteht, wobei die Leisten (19, 19') ein zwischen den beiden, das Blockiermittel bildenden Anschlagstiften (21, 21') und einem zentralen Rotationsstift (16) des gegliederten Scharniers (10) angebrachtes Federbelastungsmittel (23) aufweisen,
**dadurch gekennzeichnet, dass**
das Federbelastungsmittel (23) ein Ende aufweist, das am äußeren Ende der am zentralen Rotationsstift (16) verankerten, entgegengesetzten Leiste (19, 19') befestigt ist, während das andere Ende des Federbelastungsmittels (23) an dem mit dem Anschlagstift (21, 21') der Leiste (19, 19') verbundenen Teil befestigt ist, in dem das Federbelastungsmittel (23) untergebracht ist.

2. Gegliedertes Scharnier (10) nach Anspruch 1, **dadurch gekennzeichnet, dass** es einen ersten inneren Rundschlitz (17) zur Verstellung des Beugungswinkels sowie einen zweiten äußeren Rundschlitz (18) zur Verstellung des Verlängerungswinkels aufweist.

3. Gegliedertes Scharnier (10) nach einem der vorhergehenden Ansprüche, **dadurch gekennzeichnet, dass** jede Leiste (19, 19') eine Öffnung (20) aufweist, die dazu dient, den zentralen Stift (16) gleitend aufzunehmen.

4. Gegliedertes Scharnier (10) nach einem der vorhergehenden Ansprüche, **dadurch gekennzeichnet, dass** das aus den äußeren scheibenförmigen Elementen (11, 11') herausragende freie Längsende (24) der Leiste (19, 19') eine raue, Vorteilhafterweise gerändelte Fläche (25) aufweist.

5. Gegliedertes Scharnier (10) nach einem der vorhergehenden Ansprüche, **dadurch gekennzeichnet, dass** das Federbelastungsmittel (23) aus einer Feder besteht.

6. Gegliedertes Scharnier (10) nach einem der vorhergehenden Ansprüche, **dadurch gekennzeichnet, dass** das Federbelastungsmittel (23) in einem im Körper der Leiste (19, 19') eingeschnitten Gehäuse (30) eingesetzt ist.

7. Gegliedertes Scharnier (10) nach einem der vorhergehenden Ansprüche, **dadurch gekennzeichnet, dass** jeder Rundschlitz (17, 18) Kerben (22) aufweist, die im äußeren Teil jedes Rundschlitzes (17, 18) radial ausgerichtet sind.

8. Gegliedertes Scharnier (10) nach einem der vorhergehenden Ansprüche, **dadurch gekennzeichnet, dass** es aus einer leichten Metalllegierung und/oder einem hochwiderstandsfähigen Verbundkunststoff hergestellt ist.

9. Gegliedertes Scharnier (10) nach einem der vorhergehenden Ansprüche, **dadurch gekennzeichnet, dass** es weiterhin eine Schutzhülle enthält, die Vorteilhafterweise aus transparentem Material hergestellt ist.

10. Gegliedertes Scharnier (10) nach Anspruch 9, **dadurch gekennzeichnet, dass** die Schutzhülle so angebracht ist, dass sie um den zentralen Stift des gegliederten Scharniers rotiert.

11. Gegliedertes Scharnier (10) nach Anspruch 9 oder 10, **dadurch gekennzeichnet, dass** die Schutzhülle ein Paar auf entgegengesetzten Seiten befindliche Seitenöffnungen aufweist, welche die manuelle Betätigung der Leisten (19, 19') ermöglichen, wobei die Rotation dieser Schutzhülle den Zugang zu den Leisten (19, 19') verwehrt, so dass sie nicht manuell, versehentlich oder durch Stoßwirkung bewegt werden können.

12. Gegliedertes Scharnier (10) nach einem der vorhergehenden Ansprüche, **dadurch gekennzeichnet, dass** es an einer Knieschiene oder einer postoperativen Gehhilfe in Anwendung kommt.

## Revendications

1. Joint articulé (10) pour orthèse post-opératoire comprenant au moins un élément en forme de disque interne (13, 13'), équipé de dents de blocage (26, 26') et solidaire d'un premier montant (14), l'élément en forme de disque interne étant conçu pour se loger dans un espace délimité par une paire d'éléments en forme de disque externe (11, 11'), solidaires d'un second montant (12) et articulés avec les éléments en forme de disque internes (13, 13'), chaque élément en forme de disque externe (11) étant équipé d'une paire de fentes circonférentielles dentées (17, 18) conçues pour loger des moyens de blocage (21, 21') qui sont solidaires d'une bande (19, 19'), que l'on peut déplacer radialement et qui peut pivoter librement à l'intérieur des éléments en forme de disque externes (11), où les moyens de blocage se composent d'au moins un cheville de charnière respective (21, 21'), les bandes (19, 19') constituant un moyen de contrainte élastique (23) positionné entre la cheville de charnière respective (21, 21') formant lesdits moyens de blocage et une goupille de rotation centrale (16) du joint articulé (10), **caractérisé en ce que** ledit moyen de contrainte élastique (23) présente une extrémité fixée à l'extrémité externe de la bande opposée (19, 19') ancrée à la goupille de rotation centrale (16) tandis que l'autre extrémité du moyen de contrainte élastique (23) est fixée à la partie connectée à la cheville de charnière (21, 21') de la bande (19, 19') dans laquelle le moyen de contrainte élastique (23) est logé.

2. Joint articulé (10) selon la revendication 1, **caractérisé en ce qu'**il comprend une première fente circonférentielle interne (17), pour régler l'angle de flexion, et une seconde fente circonférentielle externe (18), pour régler l'angle d'extension.

3. Joint articulé (10) selon l'une quelconque des revendications précédentes, **caractérisé en ce que** chaque bande (19, 19') présente une ouverture (20) conçue pour loger de manière coulissante la goupille centrale. (16).

4. Joint articulé (10) selon l'une quelconque des revendications précédentes, **caractérisé en ce que** l'extrémité longitudinale libre (24) de la bande (19, 19'), dépassant des éléments en forme de disque externes (11, 11'), présente une surface rugueuse (25), avantageusement moletée.

5. Joint articulé (10) selon l'une quelconque des revendications précédentes, **caractérisé en ce que** le moyen de contrainte élastique (23) se compose d'un ressort.

6. Joint articulé (10) selon l'une quelconque des revendications précédentes, **caractérisé en ce que** le moyen de contrainte élastique (23) est introduit dans un logement (30) découpé dans le corps de la bande (19, 19').

7. Joint articulé (10) selon l'une quelconque des revendications précédentes, **caractérisé en ce que** chaque fente circonférentielle (17, 18) présente des encoches disposées radialement dans la portion externe de chaque fente circonférentielle (17, 18).

8. Joint articulé (10) selon l'une quelconque des revendications précédentes, **caractérisé en ce qu'**il est réalisé dans un alliage métallique léger et/ou un matériau plastique composite très résistant.

9. Joint articulé (10) selon l'une quelconque des revendications précédentes, **caractérisé en ce qu'**il comprend également un capot de protection, avantageusement réalisé dans un matériau transparent.

10. Joint articulé (10) selon la revendication 9, **caractérisé en ce que** le capot de protection est monté rotatif autour de la goupille centrale du joint articulé.

11. Joint articulé (10) selon la revendication 9 ou 10, **caractérisé en ce que** le capot de protection présente une paire d'ouvertures latérales opposées permettant l'activation manuelle des bandes (19, 19'), la rotation de ce capot de protection rendant les bandes (19, 19') inaccessibles, interdisant de les déplacer manuellement, par erreur ou bien à la suite d'un coup.

12. Joint articulé (10) selon l'une quelconque des revendications précédentes, **caractérisé en ce qu'**il s'applique à une orthèse de genou ou bien à un déambulateur post-opératoire.
